Europäisches Patentamt

**European Patent Office**

**Office européen des brevets**

(11) Veröffentlichungsnummer: **0 270 516 B1**

## EUROPÄISCHE PATENTSCHRIFT

(45) Veröffentlichungstag der Patentschrift: **03.04.91**

(51) Int. Cl.5: **A61K 35/16**

(21) Anmeldenummer: **87890237.8**

(22) Anmeldetag: **29.10.87**

(54) Verfahren zur Herstellung einer Faktor VIII (AHF)-hältigen Fraktion.

(30) Priorität: **03.11.86 AT 2923/86**

(43) Veröffentlichungstag der Anmeldung:
**08.06.88 Patentblatt 88/23**

(45) Bekanntmachung des Hinweises auf die
Patenterteilung:
**03.04.91 Patentblatt 91/14**

(84) Benannte Vertragsstaaten:
**AT BE CH DE ES FR GB IT LI LU NL SE**

(56) Entgegenhaltungen:
EP-A- 0 127 025
EP-A- 0 127 603
EP-A- 0 159 311
WO-A-82/04395
WO-A-86/05190

(73) Patentinhaber: **IMMUNO Aktiengesellschaft für chemisch-medizinische Produkte**
**Industriestrasse 72**
**A-1220 Wien(AT)**

(72) Erfinder: **Schwarz, Otto, Dr.**
**Celtesg. 5**
**A-1190 Wien(AT)**
Erfinder: **Linnau, Yendra, Dr.**
**Lavendelweg 24**
**A-1220 Wien(AT)**

(74) Vertreter: **Wolfram, Gustav, Dipl.-Ing.**
**Schwindgasse 7 P.O. Box 205**
**A-1041 Wien(AT)**

**Beschreibung**

Die Erfindung betrifft ein Verfahren zur Herstellung einer Faktor VIII (AHF)-hältigen Fraktion mit einer spezifischen Aktivität von mindestens 2,5 Einheiten Faktor VIII/mg Protein sowie mit einem Anteil an Immunglobulin-G (IgG) von höchstens 10 mg/1000 Einheiten Faktor VIII, bei deren therapeutischer oder prophylaktischer Anwendung das Risiko der Übertragung von viralen oder bakteriellen Infektionen vermieden bzw. weitgehend herabgesetzt ist.

Aus der Literatur ist bereits eine Mehrzahl von Verfahren zur Herstellung von Faktor VIII-Konzentraten bekannt. Diese bedienen sich als Fraktionierungsmaßnahmen einer Behandlung des Plasmas mit Äthanol, mit Äther, mit Polyäthylenglykol und/oder Glycin. Bekannt ist auch die Kryopräzipitation des Plasmas nach Pool (1965, "The New England Journal of Medicine" 273 , 1443) oder die Kryoäthanolpräzipitation des Plasmas nach Johnson (Congr. Int. Soc. Blood Transf., Sydney, Australia, Abstracts of Paper, Seite 1109 (1966)).

In der DE-A - 25 16 186 ist des weiteren ein Verfahren beschrieben, bei welchem ein aus Blutplasma gewonnenes Kryopräzipitat mazerisiert, das mazerisierte Produkt in einer Citrat-Glucose-Pufferlösung suspendiert, zentrifugiert und der so erhaltene Pufferextrakt auf einen pH-Wert im Bereich von 6,0 bis 6,8 eingestellt wird. Unter diesen Bedingungen erfolgt eine Fällung unerwünschter Verunreinigungen, worauf der den Faktor VIII enthaltende verbleibende Rückstand sterilisiert und lyophilisiert wird. Ein nach diesem Verfahren gewonnenes Faktor VIII-Produkt weist jedoch eine nur geringe spezifische Aktivität an Faktor VIII-Einheiten/mg Protein auf. Weiters ist auch der Anteil an Immunglobulin G (IgG), bezogen auf die Faktor VIII-Einheiten, unerwünscht hoch.

Ähnliche Verfahren sind auch in der AT-B - 349.639 sowie in der US-A - 4,170,639 und in der US-A - 4,104,266 beschrieben, wobei ebenfalls ausgehend von Plasma ein Kryopräzipitat gewonnen, dieses in einer Pufferlösung im neutralen pH-Bereich gelöst wird, wobei unerwünschte Proteine abgeschieden werden, der Überstand mit Aluminiumhydroxid behandelt wird, um den Prothrombinkomplex abzuscheiden, und dann die Faktor VIII enthaltende Lösung konzentriert und lyophilisiert wird. Auch bei diesen Verfahren ist die spezifische Aktivität an Faktor VIII-Einheiten/mg Protein unerwünscht gering. So beträgt die spezifische Aktivität bei der Arbeitsweise nach der US-A - 4,104,266 nach den dortigen Angaben nur 0,5 bis 0,6 Einheiten/mg Protein.

Nach der PCT-Veröffentlichung WO 82/04395 ist ein Verfahren zum Reinigen und Konzentrieren eines Faktor VIII-Komplexes bekannt, wobei die Präparation mit einer Glycin-Lösung behandelt und der Überstand mit einer Salzlösung gefällt wird.

Weiters ist gemäß der AT-B - 379 510 (der Anmelderin) ein Verfahren zur Herstellung einer Faktor VIII (AHF)-hältigen Präparation mit erhöhter spezifischer Aktivität (mindestens 1,5 Einheiten Faktor VIII/mg Protein) vorgeschlagen worden, nach welchem die Fällung unerwünschter Proteine in Anwesenheit von sulfatisiertem Polysaccharid im Neutralbereich durchgeführt und das Faktor VIII-Konzentrat aus dem Überstand durch Alkoholfällung gewonnen wird.

Aus kürzlich durchgeführten Untersuchungen (Vox Sang. 49: 319-322 (1985)) ist bekannt geworden, daß IgG-Komplexe in Faktor VIII-Konzentraten schädliche Nebenreaktionen induzieren, wie Lymphozyten-Abnormitäten sowie Änderungen des T-Helfer/T-Suppressor Zellverhältnisses in Hämophilie-Patienten.

Es hat sich gezeigt, daß die beschriebenen Verfahren zu Produkten führen, welche abgesehen von dem unerwünscht hohen IgG-Gehalt nicht genügend hitzestabil sind, um einer Inaktivierung durch Hitzebehandlung ohne erhebliche Verluste an Faktor VIII-Aktivität standzuhalten. Es besteht heute die Notwendigkeit, alle Gerinnungsfaktor-Präparationen, die aus Blutplasma hergestellt und an Patienten in großen Mengen verabreicht werden, frei von dem Risiko der Übertragung von viralen oder bakteriellen Infektionen zu halten, wobei die angewendeten Inaktivierungsverfahren in der Regel eine mehrstündige Behandlung bei Temperaturen über 60°C einschließen. Diese Forderung gilt auch für Faktor VIII-Präparationen, obgleich diese - wie bekannt -, verglichen mit anderen Gerinnungsfaktoren, relativ empfindlich und instabil sind. Ein Verfahren zur Inaktivierung von vermehrungsfähigen filtrierbaren Krankheitserregern in Blutprodukten ist beispielsweise aus der EP-A-0 159 311 bekannt.

Die Erfindung stellt sich die Aufgabe, die aufgezeigten Schwierigkeiten zu vermeiden und ein Verfahren zur Herstellung einer Faktor VIII (AHF)-hältigen Fraktion zu schaffen, bei welcher die spezifische Aktivität auf mindestens 2,5 Einheiten Faktor VIII erhöht und der Anteil an Immunglobulin G möglichst niedrig sein soll. Weiters soll die hergestellte Präparation insoweit hitzestabil sein, daß eine Inaktivierung durch Hitzebehandlung möglirh ist, ohne die Aktivität an Faktor VIII in unerwünschter Weise herabzusetzen.

Diese Aufgabe wird erfindungsgemäß durch die Kombination der folgenden Maßnahmen gelöst:
- daß aus einer Lösung einer Faktor VIII-hältigen Plasmafraktion unerwünschte Proteine in Anwesenheit von sulfatisierten Polysacchariden bei einem pH-Wert etwa im Neutralbereich gefällt und abgetrennt

EP 0 270 516 B1

werden,

- die so gereinigte Faktor VIII enthaltende Lösung mit einem Proteinfällungsmittel, ausgewählt aus Ammoniumsulfat, Ammoniumsulfat-Glycin, Natriumchlorid-Glycin, Natriumsulfat, Natriumsulfat-Natriumcitrat, Ammoniumsulfat-Natriumcitrat, Natrium chlorid-Ammonium sulfat, behandelt wird, um einen Faktor VIII enthaltenden Niederschlag auszufällen,
- der gefällte, den Faktor VIII enthaltende Niederschlag gelöst und lyophilisiert wird, und
- das Lyophilisat bei einer Temperatur und während einer Zeitdauer, die ausreichen, etwa vorhandene Viren zu inaktivieren, hitzebehandelt wird.

Es ist an sich bekannt, daß die angeführten Salze bzw. Salz-Aminosäure-Kombinationen Proteinfällungsmittel sind und als solche, wie die einleitende Übersicht des Standes der Technik zeigt, bei der Herstellung von Gerinnungsfaktor-Präparaten schon verwendet worden sind.

Indessen ist es der Vorzug der erfindungsgemäßen Kombination, daß es möglich ist, Produkte zu gewinnen, die den bisher bekannten überlegen sind, indem sie einerseits eine hohe spezifische Aktivität an Faktor VIII bei möglichst geringem Gehalt an IgG aufweisen und andererseits genügend hitzestabil sind.

Vorzugsweise wird das Lyophilisat auf einen Wassergehalt von mehr als 0,05 (5 Gew.%) und weniger als 0,70 (70 Gew.%), vorzugsweise weniger als 0,40 (40 Gew.%) eingestellt und in einem geschlossenen Behälter bei einer Temperatur im Bereich von 50 bis 121 °C unter Erhöhung des Wasserdampf-Partialdruckes behandelt.

Zweckmäßig wird die Behandlung des Lyophilisates mit Wasserdampf bei einem Druck von 0,01 bis 2 bar während einer Dauer von bis zu 100 h durchgeführt.

Als vermehrungsfähige filtrierbare Krankheitserreger kommen insbesondere Hepatitis-Viren oder HIV (Human immune deficiency virus) in Betracht.

Gemäß einer bevorzugten Ausführungsform wird die Fällung der Faktor VIII enthaltenden Lösung mit dem Proteinfällungsmittel bei einem pH-Wert von 5,6 bis 6,8 und bei einer Temperatur von 1 bis 40 °C vorgenommen.

Eine vorteilhafte Ausführungsform des erfindungsgemäßen Verfahrens ist durch die Kombination der folgenden Maßnahmen gekennzeichnet:

- daß aus der Lösung eines Kryopräzipitates in einem Citratpuffer, der gegebenenfalls Heparin, Heparinoid, eine Komplexverbindung von Heparin und Antithrombin III ("Atheplex") und/oder Aprotinin enthält, die unerwünschten Proteine bei einem pH-Wert von 6,0 bis 6,4 und einer Temperatur von 0 bis 25 °C, vorzugsweise 4 bis 8 °C, ausgefällt und abgetrennt werden,
- der gereinigte, Faktor VIII enthaltende Überstand mit einer Lösung, die Ammoniumsulfat, Ammoniumsulfat-Glycin, Natriumsulfat, Natriumsulfat-Natriumcitrat, oder Ammoniumsulfat-Natriumcitrat in einer Konzentration von 8 bis 35 % enthält, bei einem pH-Wert von 5,6 bis 6,8 behandelt wird, um einen Faktor VIII-hältigen Niederschlag auszufällen,
- und der gefällte, Faktor VIII enthaltende Niederschlag in einer Natriumchlorid-Natriumcitrat-Pufferlösung, die einen Antithrombin-Heparinoid- oder Antithrombin III-Heparinoid-Komplex sowie Albumin enthält, gelöst, ultrafiltriert oder dialysiert, lyophilisiert und durch Hitzebehandlung inaktiviert wird.

Die Erfindung wird durch die folgenden Beispiele näher erläutert:

Beispiel 1:

Aus 16,5 l Plasma wurden durch Tieffrieren und Wiederauftauen 150 g Kryopräzipitat gewonnen. Dieses wurde in 900 ml Trinatrium-Citrat-Puffer, in dem 90 mg sulfatisiertes Polysaccharid "SP 54" (Firma Benechemie), 9 Einheiten Atheplex sowie 27.000 Einheiten Aprotinin enthalten war, gelöst. Der pH-Wert der Lösung wurde auf 6,25 und die Temperatur auf 4 °C gestellt, wobei unerwünschte Proteine ausgefällt und durch Zentrifugieren abgetrennt wurden. Dem Überstand wurden Glycin und Ammoniumsulfat langsam unter Rühren bei einem pH-Wert von 6,0 bis 6,3 und bei Raumtemperatur zugesetzt, bis eine Fällungskonzentration von 120 g/l Glycin und 85 g/l Ammoniumsulfat erreicht wurden.

Der entstandene Niederschlag wurde durch Zentrifugieren abgetrennt, in einem Natriumchlorid-Citrat-Puffer gelöst und gegen den gleichen Puffer dialysiert. Zum Dialysat wurden Glycin und Albumin bis zu einer Konzentration von 10 mg/ml Glycin und 2 mg/ml Albumin zugesetzt und die Lösung lyophilisiert. Ein Teil des erhaltenen pulverförmigen Lyophilisates wurde mit Wasserdampf auf einen Feuchtigkeitsgehalt von 8 G/G % und ein anderer Teil auf einen Feuchtigkeitsgehalt von 24 bis 26 G/G % eingestellt.

Diese angefeuchteten Präparate wurden in geschlossenen Behältern unter Stickstoffatmosphäre einer Hitzebehandlung bei 60 bzw. 70 °C während einer Dauer von 10 bis 100 h unterworfen, um etwa vorhandene Viren zu inaktivieren. Anschließend wurde ihre spezifische Faktor VIII-Aktivität bestimmt. Die

3

Ergebnisse im Vergleich zum nicht hitzeinaktivierten, nach Beispiel 1 hergestellten Lyophilisat sind aus der folgenden Tabelle I zu entnehmen:

Tabelle I

| | Beispiel 1 erfindungsgemäß |
|---|---|
| Faktor VIII, Lyophilisat, nicht erhitzt | |
| spezifische Aktivität | 54,7 E/ml 100 % |
| Gehalt an IgG pro 1000 Einheiten Faktor VIII | 4,31 E/mg |
| | 1,8 mg |
| Faktor VIII, Lyophilisat mit Feuchtigkeitsgehalt 7,9 % | |
| nach 10 h 60°C erhitzt | 53,6 E/ml 98 % |
| nach 70 h 60°C erhitzt | 42,5 E/ml 78 % |
| nach 100 h 60°C erhitzt | 40,0 E/ml 73 % |
| nach 10 h 70°C erhitzt | 43,3 E/ml 79 % |
| Faktor VIII, Lyophilisat mit Feuchtigkeitsgehalt 25,2 % | |
| nach 10 h 60°C erhitzt | 45,0 E/ml 82 % |

Beispiel 2:

Um die Überlegenheit der erfindungsgemäß hergestellten Präparationen hinsichtlich der Hitzestabilität gegenüber bekannten (etwa aus der AT-B - 379.510 bekannten) zu veranschaulichen, wurde der erste Teil des Beispieles 1 wiederholt, es wurde jedoch statt der Fällung mit Glycin und Ammoniumsulfat 8 % Äthylalkohol in Anwesenheit von 1,45 Mol/l Glycin bei einem pH-Wert von 6,0 verwendet. Der Niederschlag wurde abgetrennt, in einem Citrat-NaCl-Glycin-Albumin-Puffer gelöst, lyophilisiert und das Lyophilisat wieder auf einen Feuchtigkeitsgehalt von 8 G/G % bzw. 24 bis 26 G/G % mit Wasserdampf eingestellt und, wie in Verbindung mit Beispiel 1 beschrieben, unter Stickstoffatmosphäre erhitzt. Die Ergebnisse sind aus der folgenden Tabelle II zu entnehmen, wobei einerseits der erhöhte Gehalt an IgG (30 mg gegenüber 1,8 mg) signifikant ist und andererseits bei dem zum Stand der Technik gehörenden Vergleichsbeispiel die nach der Hitzebehandlung noch vorhandenen spezifischen Aktivitäten, besonders bei längerer Erhitzung, deutlich niedriger sind.

Tabelle II

|  | Vergleichs-beispiel |
|---|---|
| Faktor VIII, Lyophilisat, nicht | |
| erhitzt | 54,3 E/ml 100 % |
| spezifische Aktivität | 2,69 E/mg |
| Gehalt an IgG pro 1000 Ein- | |
| heiten Faktor VIII | 30 mg |
| | |
| Faktor VIII, Lyophilisat mit | |
| Feuchtigkeitsgehalt 7,9 % | |
| nach 10 h 60°C erhitzt | 50,5 E/ml 93 % |
| nach 70 h 60°C erhitzt | 24,4 E/ml 45 % |
| nach 100 h 60°C erhitzt | 26,6 E/ml 49 % |
| nach 10 h 70°C erhitzt | 29,3 E/ml 54 % |
| | |
| Faktor VIII, Lyophilisat mit | |
| Feuchtigkeitsgehalt 25,2 % | |
| nach 10 h 60°C erhitzt | 24,4 E/ml 45 % |

Beispiel 3:

Der erste Teil des Beispieles 1 wurde wiederholt, zur Ausfällung des Faktor VIII-Konzentrates wurde jedoch eine Salzkombination von NaCl und Ammoniumsulfat in wässeriger Lösung verwendet, bis eine Fällungskonzentration von 10 % NaCl und 12 % Ammoniumsulfat erreicht war. Die weitere Aufarbeitung, Zentrifugieren, Lösen, Dialysieren, Lyophilisieren und Anfeuchten, erfolgte, wie in Beispiel 1 beschrieben. Die Ergebnisse sind aus der Tabelle III zu entnehmen.

Tabelle III

Faktor VIII, Lyophilisat, nicht

    erhitzt                                               42,6 E/ml 100 %

    spezifische Aktivität                            3,18 E/mg

    Gehalt an IgG pro 1000 Ein-

    heiten Faktor VIII                           9     mg

Faktor VIII, Lyophilisat mit

Feuchtigkeitsgehalt 8 %

nach 10 h 70°C erhitzt                     30    E/ml 71 %

Faktor VIII, Lyophilisat mit

Feuchtigkeitsgehalt 24,6 %

nach 10 h 60°C erhitzt                     32    E/ml 75 %

**Beispiel 4:**

Der erste Teil des Beispieles 1 wurde wiederholt, zur Ausfällung des Faktor VIII-Konzentrates wurde jedoch eine Salzkombination von Natriumcitrat und Ammoniumsulfat in wässeriger Lösung verwendet, bis eine Fällungskonzentration von 5 % Natriumcitrat und 12,5 % Ammoniumsulfat erreicht war. Die weitere Aufarbeitung, Zentrifugieren, Lösen, Dialysieren, Lyophilisieren und Anfeuchten, erfolgte, wie in Beispiel 1 beschrieben. Die Ergebnisse sind aus der Tabelle IV zu entnehmen.

Tabelle IV.

Faktor VIII, Lyophilisat, nicht

    erhitzt                                               42,2 E/ml 100 %

    spezifische Aktivität                            3,49 E/mg

    Gehalt an IgG pro 1000 Ein-

    heiten Faktor VIII                           2,6    mg

Faktor VIII, Lyophilisat mit

Feuchtigkeitsgehalt 8,3 %

nach 10 h 70°C erhitzt                     32,8 E/ml 78 %

Faktor VIII, Lyophilisat mit

Feuchtigkeitsgehalt 27,1 %

nach 10 h 60°C erhitzt                     34,5 E/ml 82 %

Beispiel 5:

Der erste Teil des Beispieles 1 wurde wiederholt, zur Ausfällung des Faktor VIII-Konzentrates wurde jedoch eine Salzlösung von Ammoniumsulfat verwendet, bis eine Fällungskonzentration von 13,2 % Ammoniumsulfat erreicht war. Die weitere Aufarbeitung, Zentrifugieren, Lösen, Dialysieren, Lyophilisieren und Anfeuchten, erfolgte, wie in Beispiel 1 beschrieben. Die Ergebnisse sind aus der Tabelle V zu entnehmen.

## Tabelle V

```
Faktor VIII, Lyophilisat, nicht
     erhitzt                              48,6 E/ml 100 %
     spezifische Aktivität                     5,70 E/mg
     Gehalt an IgG pro 1000 Ein-
     heiten Faktor VIII                        0,6   mg
```

## Fortsetzung der Tabelle V

```
Faktor VIII, Lyophilisat mit
Feuchtigkeitsgehalt 7,7 %
nach  10 h 70°C erhitzt                   28,9 E/ml 59 %


Faktor VIII, Lyophilisat mit
Feuchtigkeitsgehalt 23,7 %
nach  10 h 60°C erhitzt                   39,2 E/ml 81 %
```

In allen Fällen ist der IgG-Gehalt der erhaltenen Faktor VIII-Konzentrate weit unter 10 mg und die nach der Hitzbehandlung noch vorhandenen Restaktivitäten über 70 %.

## Ansprüche

1. Verfahren zur Herstellung einer Faktor VIII (AHF)-hältigen Fraktion mit einer spezifischen Aktivität von mindestens 2,5 Einheiten Faktor VIII/mg Protein sowie mit einem Anteil an Immunglobulin-G (IgG) von höchstens 10 mg/1000 Einheiten Faktor VIII, bei deren therapeutischer oder prophylaktischer Anwendung das Risiko der Übertragung von viralen oder bakteriellen Infektionen vermieden bzw. weitgehend herabgesetzt ist, gekennzeichnet durch die Kombination der Maßnahmen:
   - daß aus einer Lösung einer Faktor VIII-hältigen Plasmafraktion unerwünschte Proteine in Anwesenheit von sulfatisierten Polysacchariden bei einem pH-Wert etwa im Neutralbereich gefällt und abgetrennt werden,
   - die so gereinigte Faktor VIII enthaltende Lösung mit einem Proteinfällungsmittel, ausgewählt aus Ammoniumsulfat, Ammoniumsulfat-Glycin, Natriumchlorid-Glycin, Natriumsulfat, NatriumsulfatNatriumcitrat, Ammoniumsulfat-Natriumcitrat, CitratGlycin, behandelt wird, um einen Faktor VIII enthaltenden Niederschlag auszufällen,
   - der gefällte, den Faktor VIII enthaltende Niederschlag gelöst und lyophilisiert wird, und
   - das Lyophilisat bei einer Temperatur und während einer Zeitdauer, die ausreichen, etwa vorhandene Viren zu inaktivieren, hitzebehandelt wird.

2. Verfahren nach Anspruch 1, dadurch gekennzeichnet, daß das Lyophilisat auf einen Wassergehalt von mehr als 0,05 (5 Gew.%) und weniger als 0,70 (70 Gew.%), vorzugsweise weniger als 0,40 (40 Gew.%) eingestellt und in einem geschlossenen Behälter bei einer Temperatur im Bereich von 50 bis 121°C unter Erhöhung des Wasserdampf-Partialdruckes behandelt wird.

3. Verfahren nach Anspruch 1 oder 2, dadurch gekennzeichnet, daß die Behandlung des Lyophilisates mit Wasserdampf bei einem Druck von 0,01 bis 2 bar während einer Dauer von bis zu 100 h durchgeführt wird.

4. Verfahren nach einem oder mehreren der Ansprüche 1 bis 3, dadurch gekennzeichnet, daß die vermehrungsfähigen filtrierbaren Krankheitserreger Hepatitis-Viren oder HIV (Human immune deficiency virus) sind.

5. Verfahren nach einem oder mehreren der Ansprüche 1 bis 4, dadurch gekennzeichnet, daß die Fällung der Faktor VIII enthaltenden Lösung mit dem Proteinfällungsmittel bei einem pH-Wert von 5,6 bis 6,8 und bei einer Temperatur von 1 bis 40°C vorgenommen wird.

6. Verfahren nach einem oder mehreren der Ansprüche 1 bis 5, gekennzeichnet durch eine Kombination der folgenden Maßnahmen:
   - daß aus der Lösung eines Kryopräzipitates in einem Citratpuffer, der gegebenenfalls Heparin, Heparinoid, eine Komplexverbindung von Heparin und Antithrombin III und/oder Aprotinin enthält, die unerwünschten Proteine bei einem pH-Wert von 6,0 bis 6,4 und einer Temperatur von 0 bis 25°C, vorzugsweise 4 bis 8°C, ausgefällt und abgetrennt werden,
   - der gereinigte, Faktor VIII enthaltende Überstand mit einer Lösung, die Ammoniumsulfat, AmmoniumsulfatGlycin, Natriumsulfat, Natriumsulfat-Natriumcitrat, oder Ammoniumsulfat-Natriumcitrat, in einer Konzentration von 8 bis 35 % enthält, bei einem pH-Wert von 5,6 bis 6,8 behandelt wird, um einen Faktor VIII-hältigen Niederschlag auszufällen,
   - und der gefällte, Faktor VIII enthaltende Niederschlag in einer Natriumchlorid-NatriumcitratPufferlösung, die einen Antithrombin-Heparinoid- oder Antithrombin III-Heparinoid-Komplex sowie Albumin enthält, gelöst, ultrafiltriert oder dialysiert, lyophilisiert und durch Hitzebehandlung inaktiviert wird.

## Claims

1. Method for the production of a Factor-VIII-(AHF)-containing fraction having a specific activity of at least 2.5 units Factor VIII/mg protein as well as a portion of immunoglobulin G (IgG) of at most 10 mg/1000 units of Factor VIII, in whose therapeutic or prophylactic application the risk of transmissions of viral or bacterial infections is avoided or largely reduced, characterised by the combination of the measures:
   - that undesired proteins are precipitated and separated from a solution of a Factor-VIII-containing plasma fraction in the presence of sulfated polysaccharides at a pH of about in the neutral range,
   - the thus purified Factor-VIII-containing solution is treated with a protein precipitating agent selected from ammonium sulfate, ammonium sulfate glycine, sodium chloride glycine, sodium sulfate, sodium sulfate sodium citrate, ammonium sulfate sodium citrate, sodium chloride ammonium sulfate, so as to precipitate a Factor-VIII-containing precipitate,
   - the purified precipitate, containing the Factor VIII, is dissolved and lyophilised, and
   - the lyophilisate is heat treated at a temperature and for a period of time sufficient to inactivate possibly present viruses.

2. Method according to claim 1, characterised in that the lyophilisate is adjusted to a water content of more than 0.05 (5 % by weight) and less than 0.70 (70 % by weight), preferably to less than 0.40 (40 % by weight), and is treated in a closed container at a temperature in the region of from 50 to 121 centigrade while increasing the water vapour partial pressure.

3. Method according to claim 1 or 2, characterised in that the treatment of the lyophilisate with water vapour is carried out at a pressure of from 0.01 to 2 bar for a duration of up to 100 hours.

4. Method according to one or more of claims 1 to 3, characterised in that the reproductive filterable pathogens are hepatitis viruses or HIV (human immune deficiency virus).

5. Method according to one or more of claims 1 to 4, characterised in that the precipitation of the Factor-VIII-containing solution with the protein precipitating agent is carried out at a pH value of from 5.6 to 6.8 and at a temperature of from 1 to 40 centigrade.

6. Method according to one or more of claims 1 to 5, characterised by a combination of the following measures:
   - that the undesired proteins are precipitated and separated from the solution of a cryoprecipitate in a citrate buffer, optionally containing heparin, heparinoid, a complex compound of heparin and antithrombin III and/or aprotinin, at a pH of from 6.0 to 6.4 and a temperature of from 0 to 25 centigrade, preferably 4 to 8 centigrade,
   - that the purified supernatant, containing Factor VIII, is treated with a solution, containing ammonium sulfate, ammonium sulfate glycine, sodium sulfate, sodium sulfate sodium citrate or ammonium sulfate sodium citrate in a concentration of from 8 to 35 %, at a pH of from 5.6 to 6.8 %, so as to precipitate a Factor-VIII-containing precipitate,
   - and the precipitated Factor-VIII-containing precipitate is dissolved in a sodium choride sodium citrate buffer solution, containing an antithrombin heparinoid complex or an antithrombin III heparinoid complex as well as albumin, ultrafiltered or dialysed, lyophilised and inactivated by heat treatment.


## Revendications

1. Procédé de préparation d'une fraction contenant le facteur VIII (AHF) ayant une activité spécifique d'au moins 2,5 unités de facteur VIII/mg de protéines ainsi qu'une proportion d'immunoglobuline G (IgG) d'au maximum 10 mg/1 000 unités de facteur VII, dans l'emploi thérapeutique ou prophylactique de laquelle le risque de transmission d'infection virale ou bactériennes est évité ou considérablement diminué, caractérisé par l'association des mesures suivantes :
   - les protéines indésirables d'une solution de fraction plasmatique contenant le facteur VIII sont précipitées et séparées en présence de polysaccharides sulfatés à un pH se trouvant approximativement dans le domaine neutre,
   - la solution contenant le facteur VIII, ainsi purifiée, est traitée par un agent de précipitation des protéines, sélectionné parmi le sulfate d'ammonium, le sulfate d'ammonium-glycine, le chlorure de sodium-glycine, le sulfate de sodium, le sulfate de sodium-citrate de sodium, le sulfate d'ammonium-citrate de sodium, le chlorure de sodium-sulfate d'ammonium, pour donner naissance à un précipité contenant le facteur VIII,
   - le précipité contenant le facteur VIII est dissous et lyophilisé, et
   - le lyophilisat est soumis à un traitement thermique à une temperature et pendant une durée suffisantes pour inactiver les virus éventuels.

2. Procédé selon la revendication 1, caractérisé en ce que la teneur en eau du lyophilisat est ajustée à plus de 0,05 (5 % en poids) et moins de 0,70 (70 % en poids), de préférence moins de 0,40 (40 % en poids) et que le lyophilisat est traité dans un récipient fermé à une température de l'ordre de 50 à 121°C sous élévation de la pression partielle de vapeur d'eau.

3. Procédé selon la revendication 1 ou 2, caractérisé en ce que le traitement du lyophilisat par la vapeur d'eau est opéré à une pression comprise entre 0,01 et 2 bar pendant une durée allant jusqu'à 100 h.

4. Procédé selon une ou plusieurs des revendications 1 à 3, caractérisé en ce que les germes pathogènes filtrables, susceptibles de proliférer, sont des virus de l'hépatite ou des virus HIV (virus de l'immunodéficience humaine).

5. Procédé selon une ou plusieurs des revendications 1 à 4, caractérisé en ce que la précipitation de la solution contenant le facteur VIII est opérée avec l'agent de précipitation des protéines à un pH compris entre 5,6 et 6,8 et à une température de 1 à 40°C.

6. Procédé selon une ou plusieurs des revendications 1 à 5, caractérisé par l'association des mesures suivantes :

- les protéines indésirables de la solution d'un cryoprécipité dans un tampon de citrate contenant le cas échéant de l'héparine, de l'héparinoïde, un complexe d'héparine et d'antithrombine III et/ou de l'aprotinine, sont précipitées et séparées à un pH compris entre 6,0 et 6,4 et à une température située entre 0 et 25°C, de préférence entre 4 et 8°C,
- le surnageant purifié contenant le facteur VIII est traité par une solution contenant du sulfate d'ammonium, du sulfate d'ammonium-glycine, du sulfate de sodium, du sulfate de sodiumcitrate de sodium ou du sulfate d'ammonium-citrate de sodium à une concentration comprise entre 8 et 35 %, à un pH compris entre 5,6 et 6,8, pour donner naissance à un précipité contenant le facteur VIII,
- et le précipité contenant le facteur VIII est dissous dans une solution tampon de chlorure de sodium-citrate de sodium contenant un complexe antithrombine-héparinoïde ou antithrombine III-héparinoïde ainsi que de l'albumine, ultrafiltré ou dialysé, lyophilisé et inactivé par traitement thermique.